# EUROPEAN PATENT SPECIFICATION

(11) **EP 4 072 610 B1**
(45) Date of publication and mention of the grant of the patent: **30.09.2026**
(21) Application number: 20792931.6
(22) Date of filing: 06.10.2020
(51) Int. Cl.: A61M 1/16, A61M 1/34, A61M 1/36, A61M 60/113, A61M 60/247, A61M 60/37, A61M 60/438, A61M 60/837, A61M 60/851, A61M 60/279, A61M 60/109

(54) **SYSTEM FOR ULTRAFILTRATION OF BLOOD**
SYSTEM ZUR ULTRAFILTRATION VON BLUT
SYSTÈME D'ULTRAFILTRATION DU SANG

(30) Priority: 13.12.2019 SE 1951455
(43) Date of publication of application: 19.10.2022
(73) Proprietor: Gambro Lundia AB, 226 43 Lund (SE)
(72) Inventor: VARTIA, Christian, 247 64 Veberöd (SE)
(74) Representative: Sweden SHS IP Office
(86) International application number: PCT/EP2020/077917
(87) International publication number: WO 2021/115661

(56) References cited:
- EP-A1- 0 450 132
- WO-A1-2021/056091
- FR-A1- 2 734 726
- US-A- 4 684 460
- US-A1- 2009 120 864
- US-B1- 10 272 188

## Description

### Technical Field

The present disclosure relates to the field of medical treatment, particularly removal of excessive fluid, such as blood water, in a human or animal subject, also known as ultrafiltration.

### Background

Ultrafiltration (UF) encompasses a variety of membrane filtration techniques in which hydrostatic pressure forces a liquid against a semipermeable membrane.

In blood treatment, UF generally denotes a process of removing water from blood plasma. Blood is passed on a blood side of a blood filter, and a gradient of pressure is created through the semipermeable membrane. The pressure gradient forces fluid through the pores of the membrane. The pores filter electrolytes and small and middle sized molecules from the blood plasma. In contrast to the plasma, the ultrafiltrate output from the filtration pores lacks the plasma proteins and cellular components of plasma.

Fluid overload is a common problem among patients with permanent loss of kidney function, also known as chronic kidney disease (CKD). Chronic kidney disease may progress to end-stage kidney failure (CKD5), at which the patient must undergo dialysis treatment or have a kidney transplant to survive. It has been reported that more than 20% of all CKD5 patients have fluid overload as their main problem.

Even if a CKD patient has not reached end-stage kidney failure, dialysis treatment may be started to handle fluid overload in the patient. However, it is generally desirable to postpone dialysis treatment for as long as possible. Further, for the convenience of the patient and to reduce treatment cost, it is preferable to start dialysis treatment by peritoneal dialysis (PD) rather than by hemodialysis (HD). However, in CKD patients with significant fluid overload, the UF capacity of PD may not be sufficiently high. Further, CKD patients on PD may be forced to transition to HD since the UF capacity of PD may be reduced over time. Another common problem is that CKD patients on HD may have difficulties to achieve and/or maintain a given fluid status with regular treatment schedules, e.g. 2-3 treatment sessions per week, leading to large fluctuations in fluid status between treatment sessions and increased risk of intradialytic complications such as hypotension.

There is thus a general need for a simple and cost-effective system for removing excess fluid in human or animal subjects, as an alternative or supplement to dialysis treatment. Such a system may be deployed to prolong the time until dialysis treatment needs to be initiated for CKD patients, to postpone the time point when CKD patients need to switch from PD to HD, to improve the fluid balance in CKD patients between and at the onset of treatment sessions, etc.

Such a system may also be useful for treatment of patients with cardiorenal syndrome (CRS), hepatorenal syndrome (HRS), lung disorders and other insufficiencies causing fluid overload. For example, patients with congestive heart failure (CHF) are often hospitalized as a result of fluid overload.

WO2004/026364 discloses an ultrafiltration device adapted to be worn on a portion of a body of a patient. The device includes a blood filter, a blood inlet tube leading from a first blood vessel in the patient to the blood filter, and a blood outlet tube leading from the blood filter to a second blood vessel in the patient. A blood pump forces the patient's blood through the blood filter. By tailored design of the blood filter and the flow resistance downstream of the blood filter, a transmembrane pressure is created in the blood filter to cause excess fluid to separate from the blood and flow into a drain bag connected to a filtrate outlet on the blood filter. A similar device is disclosed in US2004/0054315, which also proposes to locate a dedicated UF pump between the filtrate outlet and the drain bag, and to operate the UF pump to draw excess fluid from the blood filter and thereby achieve a more precise control of the UF rate.

US2006/0122552 discloses an ultrafiltration device comprising a blood path that extends through a blood filter and includes first and second blood pumps upstream and downstream, respectively, of the blood filter. A third pump is arranged on a tubing that extends to a drain bag from a filtrate outlet on the blood filter. The pumps are controlled so that the pumping rate of the first blood pump equals the sum of the pumping rates of the second blood pump and the UF pump.

FR Al 2 734 726 relates to a hemodialysis device allowing automatic control of weight loss.

US 10,272,188 Bl provides a method of treating cerebral spinal fluid comprising: (a) utilizing a channel of a pump to draw CSF from a subject's subarachnoid space of the subject's brain or spinal column; (b) subjecting the drawn CSF to membrane dialysis against a dialysate fluid to reduce immune or inflammatory mediators; and (c) utilizing a second channel of the synchronized dual channel pump to return the dialyzed CSF to the subarachnoid space of the subject's brain or spinal column.

US 2009/120864 Al discloses a portable continuous dialysis system configured as a wearable belt in fluid communication with a separate portable unit in the form of an easy to carry bagpack or case, or a fanny pack wearable around the shoulder.

US, A, 4,684,460 relates to a hemodialysis device comprising a dialyzer, a reservoir od dialysate, a cartridge for regenerating the dialysate and a positive displacement pump with one rotor which circulates the blood and the dialysate in two separate circuits and the
conduit going from the dialysate to the dialyzer comprises a non-return valve and a pipe which is connected in by-pass downstream of said valve and which passes through a positive displacement pump with double direction of operation and which is automatically controlled by a regulation loop.

EP0450132 A1 relates to an apparatus for treating fluids in a circuit comprising a source of fluid to be treated and a receiver for the treated fluid, a fluid circuit comprising at least a first compliant fluid line (3) leading from said source to the inlet of a membrane device, a pump interconnected in said first fluid line being operative to deliver a substantially continuous fluid flow to said membrane device, a second compliant fluid line leading from the outlet of said membrane device to said receiver and at least one device being operative to create a positive flow in said fluid circuit. To increase performance of the apparatus, the device for creating a pulsatile flow is a single-roller pump operative to cause a temporary positive pressure gradient between the outlet and the inlet of the membrane device

WO2021/056091 A1 discloses an apparatus and method for semi-permeable membrane based blood filtration featuring controlled filtrate to non-filtrate separation ratio, self-regulating pressure intensification and energy recovery means and wherein basic embodiments can function without the need for valves or any other control means. These capabilities are provided for by a cooperating, positive displacement pump and motor set based apparatus wherein the greater volumetric displacement of an upstream hydraulic pump cannot be fully taken up by a lesser volumetric displacement downstream hydraulic motor as they operate synchronously within the same fluid circuit. This causes pressure intensification within that part of the fluid circuit located between the pump and motor such that a volume of fluid essentially equal to the volumetric displacement differential between the pump to motor is compelled to flow out of the circuit by passing through the pores of a semi-permeable membrane(s) located within it.

Typically, peristaltic pumps are used for pumping blood and other fluids in systems for dialysis treatment as well as in the known ultrafiltration devices above. A peristaltic pump is a positive displacement pump that comprises a moveable actuator which is operated to intermittently engage and compress a flexible tube portion to force fluid inside the tube portion to move along the tube portion. Depending on the configuration of the actuator, peristaltic pumps may be classified as linear or rotary. Peristaltic pumps offer the advantages of avoiding contact between the fluid and the actuator, having low maintenance needs, being easy to clean, inherently preventing backflow, and having a known stroke volume. However, peristaltic pumps are relatively costly and adds weight.

### Summary

It is an objective of the invention to at least partly overcome one or more of the limitations of the prior art.

In view of the foregoing, one objective is to provide a simple and cost-effective ultrafiltration system.

Another objective is to provide an ultrafiltration system which is operable at a well-defined ultrafiltration rate.

It is another objective to provide an ultrafiltration system of low weight and complexity.

These objectives are achieved by the system for ultrafiltration of blood in accordance with the appended claims.

### Brief Description of the Drawings

Embodiments will now be described herein by way of example only, with reference to the accompanying schematic drawings.
FIG. 1 is a block diagram of an ultrafiltration system according to a first embodiment according to the invention as claimed.
FIG. 2 is front view of an example of a pump head of a rotary peristaltic pump for use in accordance with embodiments.
FIG. 3A is a side view taken in direction 3A in FIG. 2 of the pump head without its backstop, FIG. 3B is a section view of a pair of line segments for the peristaltic pump, and FIG. 3C is a section view of the line segments of FIG. 3B as arranged in a pump head.
FIGS 4A (according to the invention as claimed) and 4B (not part of this invention) are block diagrams of ultrafiltration systems according to second and third embodiments.
FIG. 5A (not a part of the present invention) corresponds to FIG. 3A and is a side view of the pump head in the second embodiment of FIG. 4B (not part of this invention), and FIG. 5B (not a part of the present invention) is a section view of the line segments of FIG. 5A (not part of this invention) as arranged in a pump head.
FIG. 6 is a front view another example of a pump head of a rotary peristaltic pump for use in accordance with embodiments.
FIGS 7A-7B are top views of line segments arranged in a peristaltic pump in accordance with embodiments.
FIG. 8 (not part of this invention) is a flow chart of a method of configuring an ultrafiltration system in accordance with an embodiment.

### Detailed Description of Exemplary Embodiments

Embodiments will now be described more fully hereinafter with reference to the accompanying drawings, in which some, but not all, embodiments are shown. Indeed, the subject of the present disclosure may be embodied in many different forms and should not be construed as limited to the embodiments set forth herein; rather, these embodiments are provided so that this disclosure may satisfy applicable legal requirements. Like reference signs refer to like elements throughout.

Also, it will be understood that, where possible, any of the advantages, features, functions, devices, and/or operational aspects of any of the embodiments described and/or contemplated herein may be included in any of the other embodiments described and/or contemplated herein, and/or vice versa. In addition, where possible, any terms expressed in the singular form herein are meant to also include the plural form and/or vice versa, unless explicitly stated otherwise. As used herein, "at least one" shall mean "one or more" and these phrases are intended to be interchangeable. Accordingly, the terms "a" and/or "an" shall mean "at least one" or "one or more", even though the phrase "one or more" or "at least one" is also used herein. As used herein, except where the context requires otherwise owing to express language or necessary implication, the word "comprise" or variations such as "comprises" or "comprising" is used in an inclusive sense, that is, to specify the presence of the stated features but not to preclude the presence or addition of further features in various embodiments.

It will furthermore be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing the scope of the present disclosure. As used herein, the term "and/or" includes any and all combinations of one or more of the associated listed items.

As used herein, a "peristaltic pump" has its ordinary meaning and designates a pumping device that operates to pump a fluid through a flexible tube, which is supported on a fixed frame or backstop, by repeatedly engaging an actuator with the flexible tube. Ideally, the engagement fully compresses ("occludes") the flexible tube against the backstop. A positive displacement pumping action is produced by moving the location of the engagement location along the flexible tube. Peristaltic pumps may be subdivided into two major types, rotary or roller types, and linear or in-line types. In rotary types, the actuator comprises rollers that engage the flexible tube and move in an arc along the flexible tube on the backstop. In linear types, the actuator engages the flexible tube at right angles to the direction of flow through the flexible tube. The actuator may comprise a plurality of compressive elements, typically active, that engage the flexible tube in a defined sequence to produce the pumping action.

As used herein, a "pump head" has its ordinary meaning and designates the portion of a peristaltic pump that comprises the backstop and the actuator and may also comprise one or more motors for driving the actuator. The pump head may be configured to permit installation of the flexible tube in proper alignment with the backstop and the actuator. Alternatively, the flexible tube may be (semi-)permanently installed in the pump head and present inlet and outlet connectors for connection to external tubing.

As used herein, "ultrafiltration" (UF) has its ordinary meaning and designates a process of removing a fluid, mainly containing water, from blood plasma without substantially changing the concentration of small solutes (molecules of up to 20,000-30,000 daltons), which thus is substantially the same in the ultrafiltrate as in the plasma. As used in the present disclosure, the ultrafiltration is performed in isolation and not concurrent with dialysis treatment. This type of ultrafiltration is sometimes referred to as "isolated ultrafiltration".

Other well-known functions or constructions may not be described in detail for brevity and/or clarity. Unless otherwise defined, all terms (including technical and scientific terms) used herein have the same meaning as commonly understood by one of ordinary skill in the art to which this disclosure belongs.

The embodiments described herein relate to a system for removing excess fluid in human or animal subjects. The excess fluid is generally known in the art as "filtrate" or "ultrafiltrate", and such a system will be denoted "ultrafiltration system" hereinafter. Embodiments are based on the insight that it is possible to reduce the number of pumps in an ultrafiltration system by use of a peristaltic pump configured as a "double channel pump". Such peristaltic pumps are commercially available and are also known as "dual lumen pumps" or "double lumen pumps". This type of peristaltic pump comprises an actuator configured to concurrently engage one or more tubular elements defining two internal channels. By clever installation of such a peristaltic pump, in accordance with embodiments, it is possible to provide an ultrafiltration system with few components, and thus at a potentially reduced cost, weight and/or complexity. Embodiments are exemplified in the following for peristaltic pumps of rotary type but are equally applicable to other peristaltic pumps.

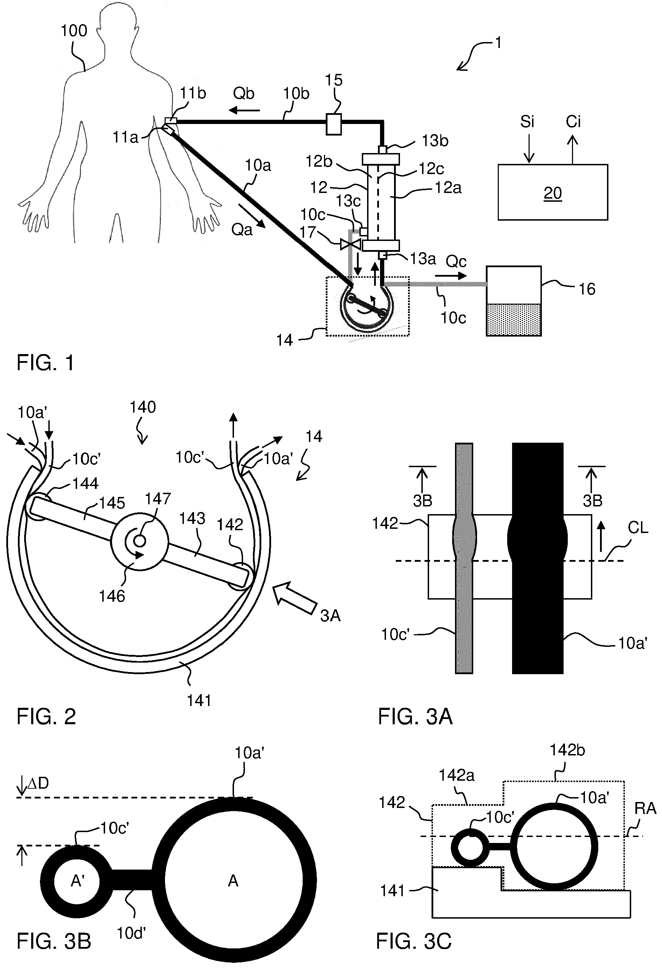

FIG. 1 illustrates an ultrafiltration system 1 in accordance with a first embodiment (according to the invention as claimed). The system 1 is configured for connection to the vascular system of a subject 100, which may be a human (as shown) or an animal. A blood circulation path is defined by a first blood line 10a, a blood compartment 12a of a blood filter 12 and second blood line 10b. The blood circulation path is configured for connection to a vascular access on the subject 100 by use of access device 11a, 11b on the ends of the first and second blood lines 10a, 10b. The vascular access may be of any known type, including but not limited to a fistula, a graft, a Scribner-shunt, or a peripheral vein on any part of the body of the subject 100. Correspondingly, the respective access device 11a, 11b may be of any known type, including but not limited to a cannula, a needle, a catheter, etc. The blood filter 12 may be any type of blood filtration device (also denoted "hemofiltration device") suitable for ultrafiltration, such as a coil dialyzer, a parallel plate dialyzer, a hollow fiber dialyzer, etc. The filter 12 comprises a housing that contains a semipermeable membrane 12c, schematically represented in FIG. 1 by a dashed line. The membrane 12c is arranged to separate the internal chamber of the housing into the blood compartment 12a and an ultrafiltrate compartment 12b. In the illustrated example, the housing comprises first and second connectors 13a, 13b, to which ends of the first and second blood lines 10a, 10b are connected to establish fluid communication through the blood compartment 12a. The respective blood line 10a, 10b may comprise flexible tubing that defines an internal fluid channel.

The system 1 further comprises an effluent line 10c, which may also comprise flexible tubing that defines an internal fluid channel and which is connected at one end to a connector 13c on the filter 12 in fluid communication with the ultrafiltrate compartment 12b. At its other end, the effluent line 10c is connected to a container or vessel 16 (as shown) or a drain. As shown, a valve 17 may be arranged in or on the effluent line 10c. The valve 17 may be configured as an on/off valve which is operable to open and close the effluent line 10c, or a restriction valve which is operable to change the flow resistance through the effluent line 10c. In one example, the valve 17 is a pinch valve or a clamp arranged to engage the outside of the effluent line 10c.

The system 1 further comprises a double-channel peristaltic pump 14, which is arranged to engage a line segment in the first blood line 10a and a line segment in the effluent line 10c.

It is to be understood that FIG. 1 is schematic and that the system 1 may comprise any number of additional devices that are commonly included in ultrafiltration or dialysis systems. For example, as indicated in FIG. 1, the second blood line 10b may comprise a drip chamber 15 configured to prevent gas (e.g. air) to be pumped with blood back to the subject 100. Further examples include an air detector (not shown) that may be arranged in or on the second blood line 10b between the drip chamber 15 and the access device 11b, and a blood leak detector (not shown) that may be arranged in or on the effluent line 10c. The system 1 may also include one or more pressure sensors (not shown) for monitoring the pressure in one or more of the lines 10a, 10b, 10c.

The system 1 further comprises an electronic control device ("controller") 20 which is configured to generate one or more control signals Ci for operative components of the system. For example, a first control signal may be generated for operating the pump 14 and a second control signal may be generated for operating the valve 17. The controller 20 may also receive and process sensor signal(s) Si from one or more sensors in the system 1, for example to implement safety functions for detecting system malfunction and/or for use when generating the control signals Ci.

In operation, when the system 1 is connected to the subject 100 and the pump 14 is active as indicated by a circular arrow in FIG. 1, blood is drawn from the subject 100, pumped in the blood circulation path and returned to the subject 100. The action of the pump 14 draws blood from the subject 100 along the first blood line 10a and drives the blood through the blood compartment 12a of the filter 12 and along the second blood line 10b. Thus, in operation, the first blood line 10a is an input line for providing blood from the subject 100 to the filter 12, and the second blood line 10b is a blood output line for returning blood to the subject 100. Since the effluent line 10c also is arranged in the pump 14, the operation of the pump 14 also draws ultrafiltrate from the ultrafiltrate compartment 12b into the effluent line 10c (grey in FIG. 1) and drives the ultrafiltrate into the container 16. As explained hereinabove, the ultrafiltrate is a liquid, mainly water, that is driven through the membrane 12c by a pressure gradient between the blood compartment 12a and the ultrafiltrate compartment 12b. The pressure gradient is created by the operation of the pump 14. In FIG. 1, Qa designates the resulting blood flow rate in the first blood line 10a, Qb designates the resulting blood flow rate in the second blood line 10b, and Qc designates the resulting ultrafiltrate flow rate ("ultrafiltration rate") in the effluent line 10c. Assuming incompressible fluids, Qc = Qa - Qb. Thus, Qc needs to be smaller than Qa, despite the fact that both flow rates are generated by the same pump 14. In one embodiment, further described below with reference to FIG. 3, Qc is set to a predefined fraction F of Qa by the configuration of the line segments 10a', 10c', F = Qc/Qa.

FIG. 2 shows an example of a double-channel peristaltic pump 14 in the system 1 of FIG. 1. Specifically, FIG. 2 is a front view of the pump head of the peristaltic pump. The pump 14 is of rotary type and the pump head 140 comprises a fixed frame 141 that defines a curved support surface for two line segments 10a', 10c', and a concentrically arranged rotor. The line segments 10a', 10c' are installed to extend in parallel along the curved support surface. The line segments 10a', 10c' are flexible tubing portions and part of or connected to the first blood line 10a and the effluent line 10c, respectively. In some implementations, the line segments 10a', 10c' may differ in structure from the other parts of the first blood line 10a and the effluent line 10c, respectively. For example, the line segments 10a', 10c' may be reinforced and/or made of thicker and/or more sturdy material to sustain the engagement forces from the pump 14 over time. In the illustrated example, the rotor comprises two rollers 142, 144 which are rotatably arranged on a respective arm 143, 145 which is fixedly arranged on a central hub 146. The rollers 142, 144 are also known as shoes, wipers or lobes. A drive shaft 147 is fixedly attached to the hub and connected for rotation by an electric motor (not shown). As the rotor turns, part of the line segment 10a', 10c' is compressed by the respective roller 142, 144 and thereby pinched closed ("occluded") so that fluid is driven along the respective line segment 10a', 10c'. Additionally, as the respective line segment 10a', 10c' opens to its natural state after the passing of the roller 142, 144 ("restitution" or "resilience"), fluid flow is induced into the pump 14. The volume of fluid pushed through the respective line segment by the respecttive roller 142, 144 is known as "stroke volume" in the art. The rotor of the pump 14 may carry more than two rollers 142, 144. When occluding the respective line segment 10a', 10c', pairs of rollers may entrap a volume of fluid, which is transported towards the pump outlet as the rollers 142, 144 rotate along the curved support surface of the frame 141.

FIG. 3A is a side view of the pump head 140 at section 3A in FIG. 2 with the frame 141 removed to illustrate the line segments 10a', 10c' and the roller 142. The roller 142 rotates in pressing engagement with the line segments 10a', 10c' in the direction of the arrow. As indicated by a bulge (exaggerated for illustrative purposes) on the respective line segment 10a', 10c', the roller 142 pushes fluid along the line segments 10a', 10c' in front of the line of contact CL between the roller 142 and the frame (not shown).

Turning to FIG. 3B, which is a section view taken at section 3B in FIG. 3A, it is seen that the line segments 10a', 10c' define internal fluid channels of different size. Specifically, the cross-sectional area A of line segment 10a' is larger than the cross-sectional area A' of line segment 10c'. The above-mentioned fraction F may be attained by simple adaptation of the line segments 10a', 10c and without requiring adaptation of the pump 14 itself. Theoretically, the above-mentioned fraction F is given by the inverse of the area ratio A/A'. In practice, other factors may modify this relation between the fraction F and area ratio A/A' to some extent, for example the degree of occlusion imparted by the pump, the compliance of line segments 10a', 10c' and/or lines 10a, 10c, the fluid pressure in lines 10a, 10c, etc. Despite such factors, the fraction F substantially corresponds to the area ratio A/A'. It is presently believed that the fraction F should be approximately 0.01-0.25 depending on implementation and desired performance of the ultrafiltration system 1. This would approximately correspond to an area ratio A/A' in the range of 4-100.

To facilitate installation of the line segments 10a', 10c' inside the pump head 140, the line segments 10a', 10c' may be formed into a unit, for example by being joined by a web portion 10d' as shown in FIG. 3B, or by other merging of the line segments 10a', 10c'. In one example, the line segments 10a', 10c' are extruded into a unitary component.

The line segments 10a', 10c' may differ significantly in outer diameter to accommodate the area ratio. The difference in outer diameter will result in a difference in height between the line segments 10a', 10c' on one or both sides of the line segments 10a', 10c' when installed in the pump head. In FIG. 3B, the height difference on one side is indicated by ΔD. It is realized that the height difference may have a negative impact on the operation of the pump 14, for example by causing a sub-optimal engagement of the rollers 142, 144 with one or both line segments 10a', 10c' against the frame 141. Such sub-optimal engagement may result in increased wear or lack of occlusion. In one embodiment, illustrated in FIG. 3C for a unitary pair of line segments, the frame 141 is provided with a support surface that matches the height difference between the line segments 10a', 10c' on the side facing the frame 141. FIG. 3C is a section view taken transverse to the extent of the line segments 10a', 10c' when installed in the pump head 140 and shows that the support surface on the frame 141 is stepped in its transverse direction so that a fictitious line between the center points of the line segments 10a', 10c' is substantially parallel to the rotation axis RA of the roller 142. As understood from FIG. 3, the rotation axis RA is defined by the attachment of the roller 142 to the arm 143. The outer perimeter of the roller 142 is correspondingly stepped and defines first and second peripheral engagement surfaces 142a, 142b for engagement with the line segments 10c', 10a', respectively, where the radius of the first engagement surface 142a is smaller than the radius of the second engagement surface 142b.

The blood lines 10a, 10b and the effluent line 10c may be provided as a set of disposables which are connected to the subject 100 and installed in an ultrafiltration machine that comprises the pump 14 and the controller 20, and optionally the container 16. Such a set of disposables in commonly denoted "line set" in the art. The set of disposables may also include the filter 12. The lines 10a, 10b, 10c and the filter 12 may be provided as separate components that are interconnected before installation, or they may be delivered as a preassembled unit. The line segments 10a', 10c' may also be part of such a set of disposables. It is conceivable that a range of sets are provided for selection by the operator of the ultrafiltration machine, where the respective set is configured to produce a specific fraction F. If the line segments 10a', 10c' are separate components for connection to the lines 10a, 10c, the line segments 10a', 10c' may be provided as a separate set of disposables.

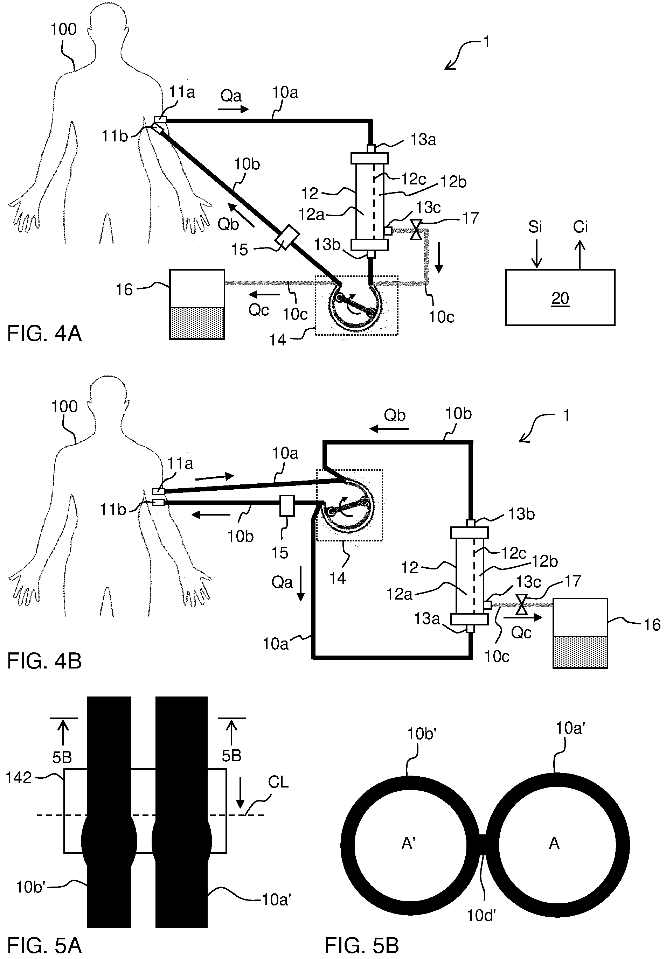

FIG. 4A ( according to the present invention) shows an ultrafiltration system 1 in accordance with a second embodiment, which differs from the first embodiment by the arrangement of the pump 14. In FIG. 4A), the pump 14 is arranged to engage a line segment in the second blood line 10b and a line segment in the effluent line 10c. For a description of the components in FIG. 4A, reference is made to the description of the first embodiment.

In operation, when the system 1 is connected to the subject 100 and the pump 14 is active as indicated by a circular arrow in FIG. 4A), blood is drawn from the subject 100, pumped in the blood circulation path and returned to the subject 100. Like in the first embodiment, the first blood line 10a is an input line for providing blood from the subject 100 to the filter 12, and the second blood line 10b is a blood output line for returning blood to the subject 100. The operation of the pump 14 also draws ultrafiltrate from the ultrafiltrate compartment 12b into the effluent line 10c and drives the ultrafiltrate into the container 16. Like in FIG. 1, Qc = Qa - Qb, where the relation between Qb and Qc may be set by the configuration of the line segments. The example presented with reference to FIGS 3A-3C is equally applicable to the embodiment in FIG. 4A (no part of this invention). To achieve a desired fraction F = Qc/Qa, the area ratio A/A' should be approximately 1/F - 1, with A' being the cross-sectional area of line segment 10c' and A being the cross-sectional area of the line segment on the second blood line 10b. For example, to achieve a fraction F = Qc/Qa in the range of 0.01-0.25, corresponding to a fraction Qc/Qb in the range of 0.01-0.33, the area ratio A/A' should be approximately in the range of 3-99.

FIG. 4B (not according to the present invention) shows an ultrafiltration system 1 in accordance with a third embodiment, which differs from the first and second embodiments by the arrangement of the pump 14. In FIG. 4B (no part of this invention), the pump 14 is arranged to engage a line segment in the first blood line 10a and a line segment in the second blood line 10b. For a description of the components in FIG. 4B (not according to the present invention), reference is made to the description of the first embodiment.

In operation, when the system 1 is connected to the subject 100 and the pump 14 is active as indicated by a circular arrow in FIG. 4B (no part of this invention), blood is drawn from the subject 100, pumped in the blood circulation path and returned to the subject 100. Like in the first embodiment, the first blood line 10a is an input line for providing blood from the subject 100 to the filter 12, and the second blood line 10b is a blood output line for returning blood to the subject 100. The pump 14 generates flow rate Qa in the first blood line 10a and flow rate Qb in the second blood line 10b. If Qa > Qb, a pressure gradient is generated over the membrane 12c, resulting in the ultrafiltration rate Qc = Qa - Qb. The relation between Qa and Qb may be set by the configuration of the line segments in the pump 14. FIG. 5A (not part of this invention) corresponds to FIG. 3A and is taken in the same direction towards the pump 14. In FIG. 5A (not part of this invention), the line segments 10a', 10b' are part of the first blood line 10a and the second blood line 10b, respectively. The bulge on the respective line segment 10a', 10b' illustrates how the roller 142 pushes fluid along the line segments 10a', 10b' in front of the line of contact CL. FIG. 5B (not part of this invention) is a section view taken at section 5B in FIG. 5A (not part of this invention) and shows that the cross-sectional area A of line segment 10a' is larger than the cross-sectional area A' of line segment 10b'. To achieve a desired fraction F = Qc/Qa, the area ratio A/A' should be approximately 1 / (1 - F). For example, to achieve a fraction F = Qc/Qa in the range of 0.01-0.25, corresponding to a fraction Qb/Qa in the range of 0.75-0.99, the area ratio A/A' should be approximately in the range of 1.01-1.33. The line segments 10a', 10b' may, like in FIG. 3B, be joined to facilitate installation, as exemplified by the web portion 10d'. Compared to the example in FIG. 3B, the line segments have more similar outer diameters, which reduces the risk for a sub-optimal engagement of the rollers 142, 144 with the line segments 10a', 10b'. However, if necessary, the modification shown in FIG. 3C may be implemented in the third embodiment (not according to the present invention) as well.

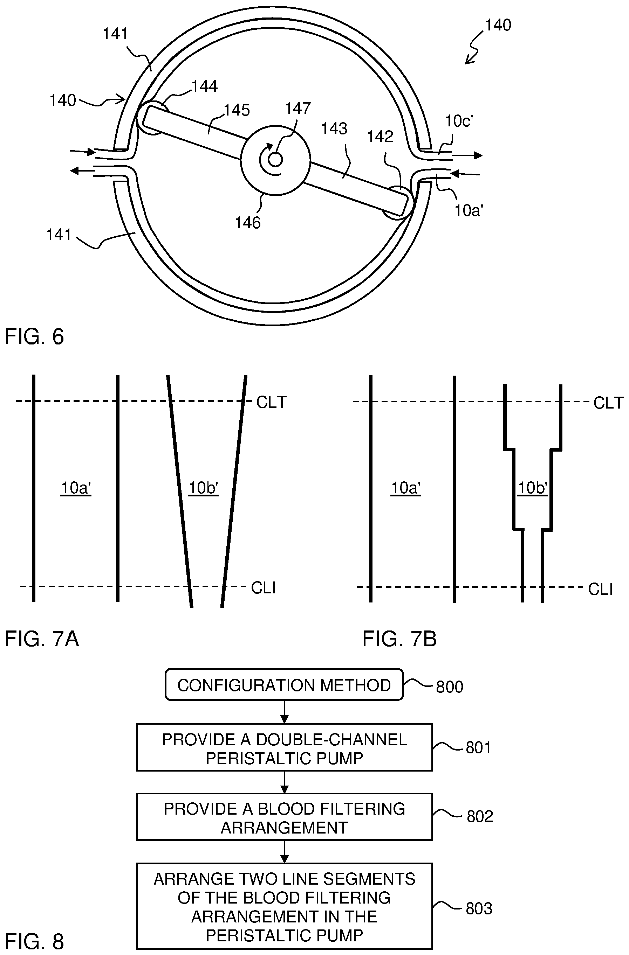

FIG. 6 illustrates an alternative configuration of the double-channel peristaltic pump 14 for use in the first, second or third embodiment. The pump 14 is of rotary type and the pump head 140 comprises a fixed frame 141 that defines two curved support surfaces, one for each line segment. The line segments are installed to extend along a respective support surface. In the illustrated example, line segments 10a', 10c' (cf. FIG. 1) are installed in the pump head 140. The pump head 140 comprises a concentrically arranged rotor of similar configuration as the rotor in FIG. 2. In contrast to the pump 14 in FIG. 2, the respective roller 142, 144 engages only one of the line segments 10a', 10c' at a time. The pump structure in FIG. 6 has the advantage of physically separating the line segments, which may facilitate a consistent engagement between the rollers 142, 144 and the line segments 10a', 10c' even if the latter differ in outer diameter.

It may be noted that the foregoing examples of area ratio between line segments presume that the line segments have a substantially uniform cross-sectional area along their extent. Embodiments are not so limited. FIG. 7A is a section view of two line segments 10a', 10b' that are arranged to extend in parallel in a pump head 140. In the illustrated example, the inner diameter of the line segment 10b' increases linearly from a contact line CLI where roller engagement is initiated to a contact line CLT where roller engagement is terminated. FIG. 7B shows another example where the inner diameter of the line segment 10b' increases stepwise along the line segment 10b' between CLI and CLT. The flow rate generated by the pump in the respective line segment 10a', 10b', and thus in the respective blood line 10a, 10b, is a function of the fluid volume between CLI and CLT, and thus a function of the total volume of the internal channel from CLI to CLT. Generally, in all embodiments described herein, the internal channel of one of the line segments in the pump 14 is dimensioned to provide a larger stroke volume than the internal fluid channel of the other line segment in the pump 14.

In the examples shown in FIG. 1 and FIGS 4A (according to the invention as claimed) and 4B (not according to the present invention), the valve 17 on the effluent line may be selectively operated by the controller 20 to restrict the effluent line 10c while the pump 14 is operated to pump fluid in the system 1. This restriction allows the momentary ultrafiltration rate to be temporarily and intermittently modified to deviate from the fraction F defined by the line segments. For example, the valve 17 be intermittently closed. The controller 21 may operate the valve 17 to attain a pre-set effective ultrafiltration rate, which represents the amount of fluid to be pumped through the effluent line 10c during a predefined time period. The controller 21 may also close the valve 17 if the ultrafiltration needs to be temporarily stopped. In such a situation, it is advantageous to keep the pump 14 running to avoid stagnant blood in the blood lines, which might cause clogging by coagulation. In the first and second embodiment, the valve 17 may be arranged between the intermediate the filter 12 and the pump 14 (FIG. 1 and FIG. 4A)). This will ensure a negative fluid pressure in the effluent line 10c downstream of the valve 17, which may minimize the risk for leaks.

FIG. 8 is a flow chart of a method 800 of configuring the ultrafiltration system 1 in accordance with any of the embodiments (not parts of the present invention) described herein. In step 801, a double-channel peristaltic pump 14 is provided, for example as part of an ultrafiltration machine. In step 802, a blood filtering arrangement is provided, for example in the form of one or more sets of disposables as discussed above. The blood filtering arrangement may thus comprise the blood lines 10a, 10b, the effluent line 10c, and the filter 12. The line segments may be integrated in two of the lines 10a, 10b, 10c, or be configured for attachment thereto. In step 803, the line segments are installed in the pump 14 in fluid communication with the two lines 10a, 10b, 10c in accordance with the first, second or third embodiment.

The ultrafiltration system 1 as described herein may be suitable for ambulatory use, by way of its potential for low weight, low cost and low complexity. In ambulatory use, the system 1 is worn or otherwise carried by the subject 100. However, the system 1 is equally suitable for stationary use, for example in a home, a dialysis clinic or a hospital. Further, the system 1 may be continuously or intermittently operated for ultrafiltration of the subject's blood in accordance with any known ultrafiltration therapy, including but not limited to acute intermittent ultrafiltration and Slow Continuous Ultrafiltration (SCUF).

The present disclosure has mainly been described above with reference to a few embodiments. However, as is readily appreciated by a person skilled in the art, other embodiments than the ones disclosed above are equally possible and the scope of the present invention is defined and limited only by the appended patent claims.

## Claims

1. A system for ultrafiltration of blood, comprising:
a blood filter (12) defining an internal chamber and comprising a semipermeable membrane (12c) arranged to separate the internal chamber into first and second compartments (12a, 12b),
a blood input line (10a) and a blood output line (10b) respectively connected to the blood filter (12) in fluid communication with the first compartment (12a),
an effluent line (10c) connected to the blood filter (12) in fluid communication with the second compartment (12b), and
a peristaltic pump (14) which is arranged for repeated engagement with a first and a second line segment and configured in accordance with a first or second segment arrangement, wherein the first segment arrangement comprises the first line segment being part of the blood input line (10a) or the blood output line (10b) and the second line segment being part of the effluent line (10c), and wherein the second segment arrangement comprises the first line segment being part of the blood input line (10a) and the second line segment being part of the blood output line (10b) **characterized in that** the system further comprises a valve (17), which is arranged in or on the effluent line (10c) and operable to selectively restrict the effluent line(10c), and wherein the peristaltic pump (14) is configured in accordance with the first segment arrangement, and wherein the valve (17) is arranged in or on the effluent line (10c) intermediate the blood filter (12) and the peristaltic pump (14).

2. The system of claim 1, wherein the first and second line segments are configured to, when the peristaltic pump (14) is operated to repeatedly engage the first and second line segments, produce a first fluid flow in the first line segment and a second fluid flow in the second line segment, wherein the first and second line segments are configured to produce the second fluid flow as a predefined fraction of the first fluid flow.

3. The system of claim 2, wherein the peristaltic pump (14) is configured in accordance with the first segment arrangement, and the predefined fraction is approximately 0.01-0.25 if the first line segment is part of the blood input line (10a), or approximately 0.01-0.33 if the first line segment is part of the blood output line (10b).

4. The system of any one of claims 2 - 3, wherein the first and second line segments are configured to, when the peristaltic pump (14) is configured in accordance with the first segment arrangement and operated to repeatedly engage the first and second line segments, produce the first fluid flow in the blood input line (10a) towards the blood filter (12) or in the blood output line (10b) away from the blood filter (12) and produce the second fluid flow in the effluent line (10c) away from the blood filter (12).

5. The system of any preceding claim, wherein the first and second line segments define a respective internal fluid channel, wherein the internal fluid channel of the first line segment is dimensioned to provide a larger stroke volume than the internal fluid channel of the second line segment when engaged by the peristaltic pump (14).

6. The system of claim 5, wherein the internal fluid channel of the first line segment has a larger cross-sectional area than the internal channel of the second line segment.

7. The system of any preceding claim, wherein the peristaltic pump (14) comprises a pump head (140) which is configured to receive the first and second line segments, wherein the pump head (140) comprises a moveable actuator (142-146) which is arranged to concurrently engage and compress the first and second line segments.

8. The system of claim 1, further comprising a control device (20), which is connected to operate the peristaltic pump (14) and to selectively operate the valve (17) during operation of the peristaltic pump (14) to cause a pre-set amount of fluid to be pumped through the effluent line (10c) during a predefined time period.

## Patentansprüche

1. System zur Ultrafiltration von Blut, umfassend:
einen Blutfilter (12), der eine Innenkammer definiert und eine semipermeable Membran (12c) umfasst, die dazu angeordnet ist, die Innenkammer in ein erstes und ein zweites Kammerabteil (12a, 12b) zu trennen,
eine Bluteingangsleitung (10a) und eine Blutausgangsleitung (10b), die jeweils mit dem Blutfilter (12) verbunden sind, der mit dem ersten Kammerabteil (12a) in Fluidverbindung ist,
eine Ablaufleitung (10c), die mit dem Blutfilter (12) verbunden ist, der mit dem zweiten Kammerabteil (12b) in Fluidverbindung ist, und
eine Peristaltikpumpe (14), die zum wiederholten Eingriff mit einem ersten und einem zweiten Leitungssegment angeordnet und in Übereinstimmung mit einer ersten oder zweiten Segmentanordnung ausgelegt ist, wobei die erste Segmentanordnung das erste Leitungssegment umfasst, das Teil der Bluteingangsleitung (10a) oder der Blutausgangsleitung (10b) ist, und das zweite Leitungssegment, das Teil der Ablaufleitung (10c) ist, und wobei die zweite Segmentanordnung das erste Leitungssegment umfasst, das Teil der Bluteingangsleitung (10a) ist, und das zweite Leitungssegment, das Teil der Blutausgangsleitung (10b) ist, **dadurch gekennzeichnet, dass** das System ferner ein Ventil (17) umfasst, das in oder an der Ablaufleitung (10c) angeordnet betreibbar ist, um die Ablaufleitung (10c) selektiv zu begrenzen, und wobei die Peristaltikpumpe (14) in Übereinstimmung mit der ersten Segmentanordnung ausgelegt ist, und wobei das Ventil (17) in oder an der Ablaufleitung (10c) zwischen dem Blutfilter (12) und der Peristaltikpumpe (14) angeordnet ist.

2. System nach Anspruch 1, wobei das erste und das zweite Leitungssegment dazu ausgelegt sind, wenn die Peristaltikpumpe (14) betrieben wird, um das erste und das zweite Leitungssegment wiederholt in Eingriff zu nehmen, einen ersten Fluidstrom in dem ersten Leitungssegment und einen zweiten Fluidstrom in dem zweiten Leitungssegment zu produzieren, wobei das erste und das zweite Leitungssegment dazu ausgelegt sind, den zweiten Fluidstrom als einen vordefinierten Teil des ersten Fluidstroms zu produzieren.

3. System nach Anspruch 2, wobei die Peristaltikpumpe (14) in Übereinstimmung mit der ersten Segmentanordnung ausgelegt ist, und der vordefinierte Teil ungefähr 0,01-0,25 beträgt, wenn das erste Leitungssegment Teil der Bluteingangsleitung (10a) ist, oder ungefähr 0,01-0,33, wenn das erste Leitungssegment Teil der Blutausgangsleitung (10b) ist.

4. System nach einem der Ansprüche 2-3, wobei das erste und das zweite Leitungssegment dazu ausgelegt sind, wenn die Peristaltikpumpe (14) in Übereinstimmung mit der ersten Segmentanordnung ausgelegt ist und betrieben wird, um das erste und das zweite Leitungssegment wiederholt in Eingriff zu nehmen, den ersten Fluidstrom in der Bluteingangsleitung (10a) in Richtung des Blutfilters (12) oder in der Blutausgangsleitung (10b) vom Blutfilter (12) weg zu produzieren, und den zweiten Fluidstrom in der Ablaufleitung (10c) vom Blutfilter (12) weg zu produzieren.

5. System nach einem vorhergehenden Anspruch, wobei das erste und das zweite Leitungssegment einen jeweiligen internen Fluidkanal definieren, wobei der interne Fluidkanal des ersten Leitungssegments dazu bemessen ist, ein größeres Schlagvolumen bereitzustellen als der innere Fluidkanal des zweiten Leitungssegments, wenn von der Peristaltikpumpe (14) in Eingriff genommen.

6. System nach Anspruch 5, wobei der interne Fluidkanal des ersten Leitungssegments eine größere Querschnittsfläche hat als der interne Kanal des zweiten Leitungssegments.

7. System nach einem vorhergehenden Anspruch, wobei die Peristaltikpumpe (14) einen Pumpenkopf (140) umfasst, der dazu ausgelegt ist, das erste und das zweite Leitungssegment aufzunehmen, wobei der Pumpenkopf (140) einen beweglichen Aktuator (142-146) umfasst, der dazu angeordnet ist, das erste und das zweite Leitungssegment gleichzeitig in Eingriff zu nehmen und zusammenzudrücken.

8. System nach Anspruch 1, ferner umfassend eine Steuervorrichtung (20), die verbunden ist, um die Peristaltikpumpe (14) zu betreiben und das Ventil (17) während des Betriebs der Peristaltikpumpe (14) selektiv zu betreiben, um zu bewirken, dass eine voreingestellte Fluidmenge während eines vordefinierten Zeitraums durch die Ablaufleitung (10c) gepumpt wird.

## Revendications

1. Système d'ultrafiltration du sang comprenant :
un filtre à sang (12) définissant une chambre interne et comportant une membrane semi-perméable (12c) agencée pour séparer la chambre interne en des premier et second compartiments (12a, 12b),
une ligne d'entrée de sang (10a) et une ligne de sortie de sang (10b) respectivement reliées au filtre à sang (12) en communication fluidique avec le premier compartiment (12a),
une ligne d'effluent (10c) reliée au filtre à sang (12) en communication fluidique avec le second compartiment (12b), et
une pompe péristaltique (14) qui est agencée pour être mise en prise de manière répétée avec un premier et un second segment de ligne et configurée conformément à un premier ou un second agencement de segment, le premier agencement de segment comprenant le premier segment de ligne qui fait partie de la ligne d'entrée de sang (10a) ou de la ligne de sortie de sang (10b) et le second segment de ligne qui fait partie de la ligne d'effluent (10c), et le second agencement de segment comprenant le premier segment de ligne qui fait partie de la ligne d'entrée de sang (10a) et le second segment de ligne qui fait partie de la ligne de sortie de sang (10b), **caractérisé en ce que** le système comprend en outre une vanne (17), qui est agencée dans ou sur la ligne d'effluent (10c) et qui peut être actionnée pour restreindre sélectivement la ligne d'effluent (10c), et la pompe péristaltique (14) étant configurée conformément au premier agencement de segment, et la vanne (17) étant agencée dans ou sur la ligne d'effluent (10c) entre le filtre à sang (12) et la pompe péristaltique (14).

2. Système selon la revendication 1, les premier et second segments de ligne étant configurés pour, lorsque la pompe péristaltique (14) est actionnée pour mettre en prise de manière répétée les premier et second segments de ligne, produire un premier écoulement de fluide dans le premier segment de ligne et un second écoulement de fluide dans le second segment de ligne, les premier et second segments de ligne étant configurés pour produire le second écoulement de fluide en tant que fraction prédéfinie du premier écoulement de fluide.

3. Système selon la revendication 2, la pompe péristaltique (14) étant configurée conformément au premier agencement de segments, et la fraction prédéfinie étant d'environ 0,01 à 0,25 si le premier segment de ligne fait partie de la ligne d'entrée de sang (10a), ou d'environ 0,01 à 0,33 si le premier segment de ligne fait partie de la ligne de sortie de sang (10b).

4. Système selon l'une quelconque des revendications 2 et 3, les premier et second segments de ligne étant configurés pour, lorsque la pompe péristaltique (14) est configurée conformément au premier agencement de segment et actionnée pour mettre en prise de manière répétée les premier et second segments de ligne, produire le premier écoulement de fluide dans la ligne d'entrée de sang (10a) vers le filtre à sang (12) ou dans la ligne de sortie de sang (10b) à distance du filtre à sang (12) et produire le second écoulement de fluide dans la ligne d'effluent (10c) à distance du filtre à sang (12).

5. Système selon l'une quelconque des revendications précédentes, les premier et second segments de ligne définissant un canal de fluide interne respectif, le canal de fluide interne du premier segment de ligne étant dimensionné pour fournir un volume de course plus grand que le canal de fluide interne du second segment de ligne lorsqu'il est en prise avec la pompe péristaltique (14).

6. Système selon la revendication 5, le canal de fluide interne du premier segment de ligne ayant une aire de section transversale plus grande que le canal interne du second segment de ligne.

7. Système selon l'une quelconque des revendications précédentes, la pompe péristaltique (14) comprenant une tête de pompe (140) qui est configurée pour recevoir les premier et second segments de ligne, la tête de pompe (140) comprenant un actionneur mobile (142-146) qui est agencé pour mettre en prise et comprimer simultanément les premier et second segments de ligne.

8. Système selon la revendication 1, comprenant en outre un dispositif de commande (20), qui est relié pour faire fonctionner la pompe péristaltique (14) et pour faire fonctionner sélectivement la vanne (17) pendant le fonctionnement de la pompe péristaltique (14) pour amener une quantité prédéterminée de fluide à être pompée à travers la ligne d'effluent (10c) pendant une période de temps prédéfinie.
